# EUROPEAN PATENT APPLICATION

(11) **EP 2 557 074 A1**
(43) Date of publication of application: **13.02.2013**
(21) Application number: 11177290.1
(22) Date of filing: 11.08.2011
(51) Int. Cl.: C07C 209/48, C07C 211/14

(54) **Process for the preparation of N,N,N',N'-tetrakis(3-aminopropyl)-1,4-butanediamine**

(71) Applicant: Astellas Pharma Inc., Tokyo 103-8411 (JP)
(72) Inventor: Ike, Kazuo, Chuo-ku Tokyo 103-8411 (JP); Watanabe, Masaru, Chuo-ku Tokyo 103-8411 (JP); Callant, Dominique, Monique, Charles, 3530 Houthalen-Helchteren (BE); Castelijns, Anna, Maria, Cornelia, Francisca, Spaubeek (NL); Dielemans, Hubertus, Johannes, Adrianus, Beek (LU); Landschützer, Herwig, 4040 Linz (AT)
(74) Representative: Beckmann, Claus

(57) **Abstract**

The invention provides a process for the preparation of *N,N,N',N'*-tetrakis(3-aminopropyl)-1,4-butanediamine by hydrogenation of 3,3',3",3"'-(1,4-butanediyldinitrilo)tetrakispropanenitrile, wherein 3,3',3",3"'-(1,4-butanediyldinitrilo)tetrakispropanenitrile is continuously added to a suspension or solution of a catalyst in a solvent which optionally comprises one or more additives and is stirred at a temperature in the range from 40 to 150 °C under a hydrogen pressure, characterized in that (i) the solvent is tetrahydrofuran, (ii) the catalyst is a hydrogenation catalyst selected from the group consisting of (1) Sponge Metal^{™} Cobalt, A-8B46, promoted with Nickel and Chromium, (2) Raney^{™} Cobalt, 2724, promoted with Chromium, (3) Actimet^{™} Cobalt and (4) Activated Ni catalyst, B 113 W, and (iii) the hydrogen pressure is from 30 to 50 bar. This special combination of catalyst and solvent allows the preparation of *N,N,N',N'*-tetrakis(3-aminopropyl)-1,4-butanediamine in high purity under a moderate hydrogen pressure which is suitable for large scale synthesis.

## Description

### TECHNICAL FIELD

The present invention relates to a process for the preparation of *N,N,N',N'*-tetrakis(3-aminopropyl)-1,4-butanediamine by hydrogenation of 3,3',3",3"'-(1,4-butanediyldinitrilo)tetrakispropanenitrile.

### BACKGROUND ART

*N,N,N',N'*-tetrakis(3-aminopropyl)-1,4-butanediamine (hereafter abbreviated as TAP-BDA) is a hexamine which can be used as complexing agent, as monomer or as core molecule for the preparation of dendrimers. It is effective as complexing agent through the possibility of bonding metal ions to the nitrogen atoms via the free electron pairs. It can be used as monomer because it can react as tetrafunctional primary amine with polyfunctional electrophiles to give polymeric structures. Especially TAP-BDA is a useful starting material of Bixalomer, which is a cross-linked polymer obtained by a reaction between TAP-BDA and 2-(chloromethyl)oxirane and useful for the treatment of hyperphosphatemia (see EP 1 682 606 A1).

Some industrially applicable synthesis methods of TAP-BDA are described in the patent literature. EP 0 707 611 A1 discloses the preparation of TAP-BDA starting from 1,4-diaminobutane through four-fold reaction with acrylonitrile to give 3,3',3",3"'-(1,4-butanediyldinitrilo)tetrakispropanenitrile (hereafter abbreviated as TPN) and subsequent hydrogenation. EP 0 382 508 A1 and US 2010/0145102 A1 disclose an improved hydrogenation method which is characterized in that a solution of TPN is continuously added over the substantially entire time of the hydrogenation reaction to a solution of a catalyst in a solvent which comprises ammonia under high hydrogen pressure, e.g. approximately 100 bar.

### DISCLOSURE OF THE INVENTION

### Problems that the Invention is to Solve

The disadvantage of the described methods is that they require high hydrogen pressure. As for a big reactor used in a large scale synthesis, there was a limit in making TPN react by high pressure. Therefore, a method of making TPN react under moderate hydrogen pressure without an increase of by-product materials was desired.

### Means for Solving the Problems

The object of the present invention was therefore to provide a novel process for the preparation of TAP-BDA, which is suitable for a large scale synthesis.

Surprisingly, it has now been found that TAP-BDA can be prepared in high purity under a moderate hydrogen pressure with the use of a special combination of a catalyst and a solvent.

The invention provides a process for the preparation of TAP-BDA by hydrogenation of TPN, wherein TPN is continuously added to a suspension or solution of a catalyst in a solvent which optionally comprises additives and is stirred at a temperature in the range from 40 to 150 °C under hydrogen pressure, characterized in that
i) the solvent is tetrahydrofuran (hereafter abbreviated as THF),
ii) the catalyst is a hydrogenation catalyst selected from the group consisting of (1) Sponge Metal^{™} Cobalt, A-8B46, promoted with Nickel and Chromium, (2) Raney^{™} Cobalt, 2724, promoted with Chromium, (3) Actimet^{™} Cobalt, (4) Activated Ni catalyst, B 113 W, and
iii) the hydrogen pressure is from 30 bar to 50 bar.

### BEST MODE FOR CARRYING OUT OF THE INVENTION

The reaction is carried out, for example, in a closed pressurized vessel which has a stirrer device. A continuous procedure in a tubular reactor is likewise conceivable.

The hydrogenation is carried out in the presence of hydrogen gas. In order to ensure a complete reduction of the nitrile groups, it is necessary to provide an adequate amount of hydrogen. Per nitrile group, at least an amount of two equivalents of hydrogen is required. The hydrogen is initially introduced in the reactor under pressure prior to the start of the reaction. In the process according to the invention, the hydrogen pressure within the reactor is maintained at from 30 to 50 bar. In one embodiment of the invention, the hydrogen pressure is from 35 to 45 bar, and in another embodiment, the hydrogen pressure is from 38 to 43 bar. The hydrogen pressure described above may be supplemented by partial pressure formed from an inert gas, such as nitrogen.

The pressure in the reactor is furthermore brought about by the presence of ammonia, if used as an additive. Ammonia serves to control the selectivity of the hydrogenation so that preferably primary amines are formed. The addition of ammonia as an additive is useful to prevent the formation of secondary and/or tertiary amines as by-products of the reaction. The molar ratio of ammonia to the starting material is preferably up to 12; in one embodiment it is between 3 and 12, and in another embodiment, it is between 4 to 8.

Further additives can likewise serve to increase the selectivity of the reaction for primary amines. For example, basic additives such as sodium hydroxide or potassium hydroxide or calcium oxide are suitable.

The temperature of the reaction is adjusted to values of from 40 to 150 °C, preferably to values of from 70 to 100 °C.

The hydrogenation is carried out in the presence of a special catalyst selected from the group consisting of
(1) Sponge Metal^{™} Cobalt, A-8B46, promoted with Nickel and Chromium, supplier: Johnson Matthey, promoters: Ni 2.9%, Cr 1.8%, A1 4.0%, and particle size: 66 µm;
(2) Raney^{™} Cobalt, 2724, promoted with Chromium, supplier: Grace, metal content: Al 2-6%, Co ≧ 86%, Fe 0-1%, Ni 1-4%, and Cr 1.5-3.5%;
(3) Actimet^{™} Cobalt, supplier: BASF, metal content: A1 5%, Co 87%, Fe 1%, Ni 4%, Cr 3%, and particle size: 35 µm;
(4) Activated Ni catalyst, B 113 W, supplier: Evonik, active metal (Ni) ≧ 90%, bulk density: 1.3 kg/1, activity: >22 ml H₂/min· g, and particle size D50: 53 µm.

These catalysts are known to the person skilled in the art and commercially available from several manufacturers. One embodiment of the catalyst is Activated Ni catalyst, B 113 W. Because of the cost, waste handling, and the possibility to leave out of NH₃ during the reduction, (4) Activated Ni catalyst, B 113 W is a preferred catalyst.

Catalysts are often supplied as aqueous suspension. Within the context of the invention, the catalyst can be used either water-moist, or else the water in the supply form is exchanged for THF by washing.

According to the invention, the optionally washed catalyst is initially introduced into the reactor, together with THF, hydrogen and optionally one or more additives, e.g. ammonia and/or sodium hydroxide, potassium hydroxide or calcium oxide, and stirred under the reaction conditions.

The amount of catalyst used is initially dependent on the geometry and the handling of the reactor used. According to the invention, an amount of dry catalyst of 2.5-20% by weight relative to the amount of TPN suffices to ensure complete conversion of TPN with almost complete selectivity in favor of TAP-BDA. In one embodiment the amount of dry catalyst is 2.5-13% by weight of TPN; in another embodiment it is 7.5-13%, and in yet another embodiment it is 5-15% or 9-12%.

The content of the reactor must be carefully mixed so that both sufficient hydrogen is introduced into the reaction mixture and the catalyst is adequately circulated in order to be able to interact with TPN. For this, stirring devices within the reactor are suitable.

The reaction is started by continuous addition of TPN into the reactor against the hydrogen pressure at a rate which is not greater than the maximum rate at which the TPN is reacting with hydrogen. In a preferred embodiment, TPN is added as a solution in a solvent. The concentration of TPN should be selected so that a suitable feed rate and residence time can be set. Preferably, from 10 to 50% by weight of TPN is mixed with the solvent, e.g. THF. In a preferred embodiment, the solution of TPN is continuously added to a suspension or solution of the catalyst by a pump. The hydrogenation reaction continues efficiently by the continuous addition of TPN solution at an appropriate feed rate. Usually, the addition of TPN is carried out within a period from 2 hours to 24 hours, preferably within from 4 hours to 24 hours. In another embodiment it is carried out within a period from 6 hours to 21 hours. When the addition of TPN is complete, the continued absorption of hydrogen can occur, caused by the reaction which has not yet completely concluded. When hydrogen absorption has finished, the reaction is ended. Typical post-reaction time is between 10 minutes and 4 hours, usually around 1 hour.

After the reaction, the catalyst used is filtered off from the reaction mixture. This can take place via a filter unit outside of the reactor or by filter candles and a riser tube inside the vessel. Thus, the catalyst can either be fed to a suitable disposal or else to recycling. For the recycling, the catalyst can either remain in the reactor and be used again immediately, or else it is reactivated prior to the next use. For the reactivation, the catalyst can, for example, be washed with an aqueous solution of NaOH or KOH at elevated temperature and be afterwashed with water until the solution which runs off is almost neutral.

TAP-BDA manufactured by the production method of the invention is pure enough and useful as the manufacturing raw material of dendrimers or cross-linked polymers. For example, TAP-BDA of the invention is a useful starting material of a cross-linked polymer obtained by a reaction between TAP-BDA and 2-(chloromethyl)oxirane, e.g. Bixalomer. Such a cross-linked polymer can be prepared according to a protocol described in Example 9 of EP 1 682 606 A1.

### EXAMPLES

### Example 1

A catalyst screening was performed in a 100 ml Parr autoclave (made of Hastelloy C) equipped with a turbine stirrer and a Gilson pump, for the addition of TPN solution.

A 30 wt% TPN solution in THF was prepared. A cobalt catalyst (as a 50%wet catalyst, 3.65 g) suspension in THF (32 g) was added to the open reactor. After closing the reactor, the reaction mixture was purged 10 times with 3 bar N₂ and 3 times with 40 bar H₂, and then NH₃ (5.5-6.0 g) was added via a dosing barrel to the reactor. The mixture was heated to 50 °C and the pressure in the reactor was increased with H₂ to 45 bar. The 30 wt% TPN solution (57 g) was dosed during 4 hours via a dosing barrel to the reactor. After dosing, reaction mixture was stirred overnight at 45 bar, then cooled to < 30 °C, depressurized and purged 3 times with 3 bar N₂ with stirring. After filtration, the solution was washed with 100 ml THF and concentrated in vacuo at 45 °C by rotary evaporator. The oil was analyzed via ¹H-NMR to determine the conversion of TPN, and via HPLC to determine the amine side products.

The following catalysts were used:
(1) Sponge Metal^{™} Cobalt, A-8B46, promoted with Nickel and Chromium, supplier: Johnson Matthey
(2) Raney^{™} Cobalt, 2724, promoted with Chromium, supplier: Grace
(3) Actimet^{™} Cobalt, supplier: BASF

The following comparison catalysts were used:
(a) Cobalt, B2112Z, 2008-135, supplier: Evonik
(b) Cobalt Grade 3100, 151009 Lab, supplier: CAT Alloy
(c) Activated Alloy Cobalt, B0910-0H03-0313, supplier: Monarch

Results are shown in Table 1. Under mild H₂ pressure of 45 bar, catalysts (1) - (3) performed complete conversion with no or little by-product materials which could be determined with HPLC. On the other hand, comparison catalysts (a) gave poor conversion, and (b) and (c) gave by-product materials that exceeded 1%.

**Table 1**

| Exp. No. | Catalyst | | Conversion (%) | TAP-BDA area % | By-product materials | | | |
|---|---|---|---|---|---|---|---|---|
| | | | | | 1-arm area % | 2-arm area % | 3-arm area % | unknown area % |
| 72 | Cobalt | (1) | 100.0 | 100.0 | <0.1 | <0.1 | <0.1 | <0.1 |
| 71* | | (2) | 100.0 | 100.0 | <0.1 | <0.1 | <0.1 | <0.1 |
| 74 | | (3) | 100.0 | 99.8 | <0.1 | <0.1 | 0.2 | <0.1 |
| 73 | | (a) | ~25 | 49.1 | <0.1 | 9.5 | 14.5 | 26.9 |
| 75 | | (b) | 100.0 | 98.7 | <0.1 | <0.1 | 0.2 | 1.3 |
| 76 | | (c) | 100.0 | 96.3 | <0.1 | 0.4 | 3.0 | 0.3 |

| | | | | | | | | |
|---|---|---|---|---|---|---|---|---|
| * The reaction time was 17 hours. 1-arm: *N*-(3-aminopropyl)-1,4-butanediene (CAS: 124-20-9) 2-arm: *N,N'*-bis(3-aminopropyl)-1,4-butanediene (CAS: 71-44-3) 3-arm: *N,N,N'*-tris(3-aminopropyl)-1,4-butanediene (CAS: 154263-92-0) | | | | | | | | |

### Example 2

The procedures of Example 1 were repeated, except that 50 wt% KOH-water solution (10 wt%) were added to a Ni catalyst suspension in THF in the open reactor, and the hydrogenation reaction was run at 80 °C.

The following catalyst was used:
(4) Activated Ni catalyst, B 113 W, supplier: Evonik

The following comparison catalysts were used:
(d) Activated Ni catalyst, BLM 112 W, supplier: Evonik
(e) Activated Ni catalyst, Activat 1600, supplier: CAT Alloy

Results are shown in Table 2. Under mild H₂ pressure of 45 bar, Ni catalyst (4) performed complete conversion with no by-product materials which could be determined with HPLC. On the other hand, comparison catalyst (e) gave poor conversion, and comparison catalyst (d) seems to be less reactive because of the formation of more side product (3-arm). Hydrogenation was performed completely with catalyst (4) with little by-product materials in larger (10 ltr.) scale production (Exp. No. 88).

**Table 2**

| Exp. No. | Catalyst | | Conversion (%) | TAP-BDA area % | By-product materials | | | |
|---|---|---|---|---|---|---|---|---|
| | | | | | 1-arm area % | 2-arm area % | 3-arm area % | unknown area % |
| 77 | Nickel | (4) | 100.0 | 100.0 | <0.1 | <0.1 | <0.1 | <0.1 |
| 88** | | (4) | 100.0 | 99.97 | <0.1 | <0.1 | 0.03 | <0.1 |
| 78*** | | (d) | 100.0 | 100.0 | <0.1 | <0.1 | <0.1 | <0.1 |
| 86*** | | (d) | 100.0 | 97.3 | <0.1 | <0.1 | 2.98 | <0.1 |
| 85 | | (e) | low | low | | | | |

| | | | | | | | | |
|---|---|---|---|---|---|---|---|---|
| ** 10 ltr. scale production *** executed twice under the same condition | | | | | | | | |

### Example 3

The procedures of Example 1 were repeated, except that 50 wt% KOH-water solution (10 wt%) were added to a Ni catalyst suspension in THF in the open reactor, NH₃ was not added in the reactor, and the hydrogenation reaction was run at 80 °C.

The following catalyst was used:
(4) Activated Ni catalyst, B 113 W, supplier: Evonik

The following comparison catalysts were used:
(d) Activated Ni catalyst, BLM 112 W, supplier: Evonik
(e) Activated Ni catalyst, Activat 1600, supplier: CAT Alloy
(f) Raney^{™} Ni, 2400, supplier: Grace
(g) Activated Ni catalyst, Activat 1200, supplier: CAT Alloy
(h) Activated Ni catalyst, MC700, supplier: Evonik

Results are shown in Table 3. Under mild H₂ pressure of 45 bar and without addition of NH₃, catalysts (4) performed complete conversion without by-product materials which could be determined with HPLC. On the other hand, all comparison catalysts gave poor conversion.

**Table 3**

| Exp. No. | Catalyst | | Conversion (%) | TAP-BDA area % | By-product materials | | | |
|---|---|---|---|---|---|---|---|---|
| | | | | | 1-arm area % | 2-arm area % | 3-arm area % | unknown area % |
| 79*** | Nickel | (4) | 100.0 | 100.0 | <0.1 | <0.1 | <0.1 | <0.1 |
| 82*** | | (4) | 100.0 | 99.73 | <0.1 | <0.1 | 0.27 | <0.1 |
| 81 | | (d) | low | low | | | | |
| 84 | | (e) | low | low | | | | |
| 80 | | (f) | low | low | | | | |
| 83 | | (g) | low | low | | | | |
| 87 | | (h) | low | low | | | | |

| | | | | | | | | |
|---|---|---|---|---|---|---|---|---|
| *** executed twice under the same conditions | | | | | | | | |

### Example 4

For the reduction of TPN to TAP-BDA, the hydrogenation was performed in a large reactor (12 m³) at a maximum pressure of 45 bar.

A 30 wt% TPN solution in THF was prepared. 50 wt% KOH solution (20 kg) and activated Ni catalyst, B 113 W (170 kg) were mixed, washed with 15 L water, then THF (500 kg) was added to make a catalyst solution. The catalyst solution was added to the large reactor which contained THF (3,000 kg), then the mixture was washed with THF (1,000 kg). After addition of NH₃ (450 kg) to the reactor, the mixture was heated to 80 °C, then the pressure in the reactor was increased with H₂ to 38 - 43 bar.

The 30 wt% TPN solution (5,000 kg) was dosed during 12 hours to the reactor while the reaction mixture was stirred. After dosing, reaction mixture was stirred for another 1 hour, cooled to < 50 °C, filtrated and concentrated in vacuo to obtain TAP-BDA the purity of which was over 98%.

## Claims

1. Process for the preparation of *N,N,N',N'*-tetrakis(3-aminopropyl)-1,4-butanediamine by hydrogenation of 3,3',3",3"'-(1,4-butanediyldinitrilo)tetrakispropanenitrile, wherein 3,3',3",3"'-(1,4-butanediyldinitrilo)tetrakispropanenitrile is continuously added to a suspension or solution of a catalyst in a solvent which optionally comprises one or more additives and is stirred at a temperature in the range from 40 to 150 °C under a hydrogen pressure, **characterized in that**
i) the solvent is tetrahydrofuran,
ii) the catalyst is a hydrogenation catalyst selected from the group consisting of (1) Sponge Metal^{™} Cobalt, A-8B46, promoted with Nickel and Chromium, (2) Raney^{™} Cobalt, 2724, promoted with Chromium, (3) Actimet^{™} Cobalt and (4) Activated Ni catalyst, B 113 W, and
iii) the hydrogen pressure is from 30 to 50 bar.

2. The process according to claim 1, **characterized in that** ammonia is added as an additive.

3. The process according to claims 1 or 2, **characterized in that** the amount of catalyst used, calculated on a dry catalyst basis, is 2.5-13% by weight of the amount of 3,3',3",3"'-(1,4-butanediyldinitrilo)tetrakispropanenitrile used.

4. The process according to one of claims 1 to 3, **characterized in that** the catalyst is Activated Ni catalyst, B 113 W.

5. The process according to one of claims 1 to 4, **characterized in that** the hydrogen pressure is from 35 to 45 bar.

6. The process according to one of claims 1 to 5, **characterized in that** sodium hydroxide or potassium hydroxide is added as an additive.

7. The process according to one of claims 1 to 6, **characterized in that** the temperature is between 70 and 100 °C.
